# EUROPEAN PATENT APPLICATION

(11) **EP 3 305 277 A1**
(43) Date of publication of application: **11.04.2018**
(21) Application number: 16803165.6
(22) Date of filing: 25.05.2016
(51) Int. Cl.: A61K 8/81, A61Q 3/02

(54) **NAIL COSMETIC AND NAIL ART KIT**

(30) Priority: 29.05.2015 JP 2015109326
(71) Applicant: FUJIFILM Corporation, Tokyo 106-8620 (JP)
(72) Inventor: ISHIJI, Yohei, Haibara-gun Shizuoka 421-0396 (JP); OOHASHI, Hidekazu, Haibara-gun Shizuoka 421-0396 (JP)
(74) Representative: Hoffmann Eitle
(86) International application number: PCT/JP2016/065440
(87) International publication number: WO 2016/194730

(57) **Abstract**

An object of the present invention is to provide a nail cosmetic material having excellent adhesiveness of a cured product and coatability and also having excellent peelability, and a nail art kit containing the nail cosmetic material.

The nail cosmetic material of the present invention including: hydroxyethyl acrylamide as a component 1; and at least one compound selected from the group consisting of dimethyl acrylamide, diethyl acrylamide, acryloyl morpholine, and butoxy diethylene glycol methacrylate, as a component 2.

## Description

### BACKGROUND OF THE INVENTION

### 1. Field of the Invention

The present invention relates to a nail cosmetic material and a nail art kit.

### 2. Description of the Related Art

In recent years, the popularity of nail art that designs nails of the hands or feet is increasing, and a technology for forming artificial nails (fake nails, nail tips, and the like) made of synthetic resins on nails (natural nails) has been developed. Particularly, recently, a nail decoration called an artificial nail, which is used by applying a gel-like decorative curable composition containing a urethane resin and a photopolymerizable monomer on the nail followed by curing the composition through irradiation of the composition with ultraviolet rays, is attracting attention due to reasons that the nail decoration provides a clear finish, is long-wearing and highly adhesive to the nail, and is odorless as in a case of an acrylic resin.

Artificial nails and compositions disclosed in JP2004-275736A, JP2002-505915U, and JP4981184B are known as artificial nails and compositions (nail cosmetic materials) that can be used for forming the artificial nails, in the related art.

In JP2004-275736A, an artificial nail which fits various sizes and shapes of fingers includes: (a) a main body of a polymer which has an upper surface and a lower surface and has a shape of a nail; and (b) a layer made of a deformable material which is attached to at least a part of the lower surface, in which the artificial nail fits the upper surface of a natural nail in a case of being attached to the natural nail is disclosed.

In JP2002-505915U, a liquid artificial nail composition containing one or more addition polymerizable ethylenically unsaturated monomers, in which the composition further contains a monoethylenically unsaturated vinyl monomer containing two or more carbonyl groups is disclosed.

In JP4981184B, a base coat for a photocurable gel nail which contains the following (A) to (F) is disclosed.
(A) Polyether skeleton urethane methacrylate oligomer: 100 parts by weight, (B) alicyclic (meth)acrylate monomer: 20 to 60 parts by weight, (C) acrylamide monomer which is a liquid at 25°C: 5 to 20 parts by weight, (D) bifunctional (meth)acrylate monomer having ethylene oxide in a molecule: 20 to 40 parts by weight, (E) polyfunctional monomer such as tri- or higher-functional monomer: 0 parts by weight or 0 to 1 parts by weight (where 0 parts by weight is not included), (F) photopolymerization initiator: 5 to 20 parts by weight

### SUMMARY OF THE INVENTION

An object of the present invention is to provide a nail cosmetic material having excellent adhesiveness of a cured product and coatability and also having excellent peelability, and a nail art kit containing the nail cosmetic material.

The object is achieved by means described in the following <1> or <11>. The object will be shown below together with <2> to <10> which are preferred embodiments.
<1> A nail cosmetic material comprising: hydroxyethyl acrylamide as a component 1; and at least one compound selected from the group consisting of dimethyl acrylamide, diethyl acrylamide, acryloyl morpholine, and butoxy diethylene glycol methacrylate, as a component 2.
<2> The nail cosmetic material according to <1>, in which a component 2 includes at least two compounds selected from the group consisting of dimethyl acrylamide, diethyl acrylamide, acryloyl morpholine, and butoxy diethylene glycol methacrylate.
<3> The nail cosmetic material according to <1> or <2>, further comprising a photopolymerization initiator.
<4> The nail cosmetic material according to any one of <1> to <3>, further comprising a polymer and/or an oligomer.
<5> The nail cosmetic material according to <4>, in which the polymer and/or the oligomer are a polymer and/or an oligomer having a urethane bond.
<6> The nail cosmetic material according to <4> or <5>, in which the polymer and/or the oligomer have a (meth)acryloyl group.
<7> The nail cosmetic material according to any one of <4> to <6>, in which the content of the polymer and/or the oligomer is 10 to 60 parts by mass with respect to 100 parts by mass of the nail cosmetic material.
<8> The nail cosmetic material according to any one of <1> to <7>, in which the content of the component 1 is 1 to 25 parts by mass with respect to 100 parts by mass of the nail cosmetic material.
<9> The nail cosmetic material according to any one of <1> to <8>, in which the content of the component 2 is 1 to 50 parts by mass with respect to 100 parts by mass of the nail cosmetic material.
<10> The nail cosmetic material according to any one of <1> to <9>, further comprising a solvent.
<11> A nail art kit comprising the nail cosmetic material according to any one of <1> to <10>.

According to the present invention, it is possible to provide a nail cosmetic material having excellent adhesiveness of a cured product and coatability and also having excellent peelability, and a nail art kit containing the nail cosmetic material.

### DESCRIPTION OF THE PREFERRED EMBODIMENTS

Hereinafter, the present invention will be described in detail.

In the present specification, the description "xx to yy" represents a numerical range including xx and yy. In addition, "hydroxyethyl acrylamide" or the like is also simply referred to as a "component 1".

In addition, in the present invention, "mass%" has the same meaning as that of wt%" and "parts by mass" has the same meaning as that of "parts by weight".

In addition, in the present invention, a combination of two or more preferable embodiments is a more preferable embodiment.

In the present specification, regarding description of a group in a compound represented by a formula, in a case where there is no description as to whether the group is substituted or unsubstituted, the group also contains a group having a substituent as well as an unsubstituted group unless otherwise specified in a case where the group can further have the substituent. For example, in the description of a formula, in a case where there is a description that "R represents an alkyl group, a cycloalkyl group, or an aryl group", the description means that "R represents an unsubstituted alkyl group, a substituted alkyl group, an unsubstituted cycloalkyl group, a substituted cycloalkyl group, an unsubstituted aryl group, or a substituted aryl group". In addition, in the present specification, (meth)acryl indicates a concept including both acryl and methacryl, and the same also applies to (meth)acrylate, (meth)acrylic acid, (meth)acrylamide, and the like.

In addition, a polymer in the present specification includes a copolymer.

### (Nail Cosmetic Material)

The nail cosmetic material of the present invention includes: hydroxyethyl acrylamide as a component 1; and at least one compound selected from the group consisting of dimethyl acrylamide, diethyl acrylamide, acryloyl morpholine, and butoxy diethylene glycol methacrylate, as a component 2.

The present inventors and the like have conducted detailed studies, and as a result, the present inventors have found that the nail cosmetic material of the present invention containing the component 1 and the component 2 has excellent adhesiveness of a cured product and coatability and has excellent peelability.

Although the detailed mechanism by which the effects are obtained is unknown, it is estimated that a concerted effect is exhibited by combining the component 1 having high hydrogen bonding properties and hydrophilicity and having characteristics of being a liquid at 25°C with the component 2 which is highly compatible with the component 1 and is a low viscosity monomer.

The nail cosmetic material of the present invention can be suitably used not only as a composition for forming a gel nail or nail polish such as manicure or pedicure, but also as a nail protective agent such as a nail coat.

The nail cosmetic material of the present invention can suitably be used for any of a primer layer, a base layer, a color layer, and/or a top layer in an artificial nail. Among them, the nail cosmetic material of the present invention can suitably be used as a nail cosmetic material for a primer layer or a base layer and more suitably used as a nail cosmetic material for a base layer.

In addition, the nail cosmetic material of the present invention is preferably a photocurable composition.

### <Component 1: Hydroxyethyl Acrylamide>

The nail cosmetic material of the present invention contains hydroxyethyl acrylamide as a curable component.

It is estimated that it is possible to improve an adhesive force to a nail due to high hydrogen bonding properties, high hydrophilicity, and characteristics of being a liquid at 25°C of the hydroxyethyl acrylamide which is contained in the nail cosmetic material of the present invention.

### [Content of Component 1]

The content of hydroxyethyl acrylamide in the nail cosmetic material of the present invention is, with respect to 100 parts by mass of the nail cosmetic material, preferably 0.5 to 35 parts by mass, more preferably 1 to 25 parts by mass, and still more preferably 3 to 15 parts by mass.

In a case where the nail cosmetic material contains hydroxyethyl acrylamide within the above-described ranges, it is possible to achieve both high coatability of the nail cosmetic material and improved adhesiveness of a cured product.

### <Component 2: at least One Compound Selected from Group Consisting of Dimethyl Acrylamide, Diethyl Acrylamide, Acryloyl Morpholine, and Butoxy Diethylene Glycol Methacrylate>

The nail cosmetic material of the present invention contains at least one compound selected from the group consisting of dimethyl acrylamide, diethyl acrylamide, acryloyl morpholine, and butoxy diethylene glycol methacrylate, as the component 2.

Among them, the nail cosmetic material of the present invention preferably contains at least one compound selected from the group consisting of dimethyl acrylamide and acryloyl morpholine, as the component 2 from the viewpoint of adhesiveness of a cured product.

Although the reason is unclear, the component 2 is a low viscosity monomer which is highly compatible with hydroxyethyl acrylamide, and therefore, it is estimated that the component 2 has an effect of promoting penetration of hydroxyethyl acrylamide into a nail and improving the adhesiveness.

In addition, the nail cosmetic material of the present invention preferably contains at least two compounds selected from the group consisting of dimethyl acrylamide, diethyl acrylamide, acryloyl morpholine, and butoxy diethylene glycol methacrylate, as components 2.

In a case where the nail cosmetic material contains two or more components 2 as described above, it is preferable that the nail cosmetic material contains at least dimethyl acrylamide or at least acryloyl morpholine and butoxy diethylene glycol methacrylate.

According to the embodiment, it is possible to obtain a nail cosmetic material having excellent coatability, and adhesiveness and peelability of a cured product.

### [Content of Component 2]

It is preferable that the content of the component 2 in the nail cosmetic material of the present invention is, with respect to 100 parts by mass of the nail cosmetic material, within a range of 1 to 50 parts by mass and more preferably 5 to 25 parts by mass.

By adding the component 2 to the nail cosmetic material within the above-described ranges, it is possible to obtain a nail cosmetic material having high coatability. It is estimated that the coatability is improved due to reduced viscosity derived from high hydrogen bonding properties of hydroxyethyl acrylamide.

### <Other Ethylenically Unsaturated Compounds>

The nail cosmetic material of the present invention may contain ethylenically unsaturated compounds other than hydroxyethyl acrylamide, dimethyl acrylamide, diethyl acrylamide, acryloyl morpholine, and butoxy diethylene glycol methacrylate.

Specific examples of the ethylenically unsaturated compounds include (meth)acrylic acid; monofunctional (meth)acrylic acid esters such as methyl (meth)acrylate, ethyl (meth)acrylate, 2-ethyl hexyl (meth)acrylate, 2-hydroxyethyl (meth)acrylate, 2-hydroxypropyl (meth)acrylate, glycerin monomethacrylate, and isobornyl (meth)acrylate; N-vinyl amides such as N-vinyl pyrrolidone and N-vinyl caprolactam; styrenes such as styrene, 4-acetoxystyrene, 4-carboxystyrene, and the like; polyfunctional (meth)acrylic acid esters such as ethylene glycol di(meth)acrylate, triethylene glycol di(meth)acrylate, tetraethylene glycol di(meth)acrylate, propylene glycol di(meth)acrylate, trimethylolpropane tri(meth)acrylate, pentaerythritol tri(meth)acrylate, pentaerythritol tetra(meth)acrylate, dipentaerythritol penta(meth)acrylate, dipentaerythritol hexa(meth)acrylate, 1,6-hexanediol di(meth)acrylate, cardo epoxy di(meth)acrylate, neopentyl glycol di(meth)acrylate; urethane (meth)acrylate including diisocyanate- and hydroxyl group-containing (meth)acrylic acid derivatives and optionally including diols.

In addition, it is preferable that the nail cosmetic material of the present invention does not contain methacrylamide or diacetone acrylamide as a curable component.

Among the ethylenically unsaturated compounds, isobornyl acrylate, isobornyl methacrylate, acrylic acid, methacrylic acid, N-vinyl pyrrolidone, N-vinyl caprolactam, 2-hydroxyethyl methacrylate, 2-hydroxypropyl methacrylate, 2-hydroxybutyl methacrylate, and glycerin monomethacrylate are preferable and isobornyl acrylate, isobornyl methacrylate, acrylic acid, 2-hydroxyethyl methacrylate, and 2-hydroxypropyl methacrylate are more preferable.

The ethylenically unsaturated compounds may be used singly or in combination of two or more thereof.

The content of the other ethylenically unsaturated compounds in the nail cosmetic material of the present invention is, with respect to 100 parts by mass of the nail cosmetic material, preferably 0 parts by mass to 50 parts by mass, more preferably 5 to 40 parts by mass, and still more preferably 10 to 30 parts by mass.

In a case where the content of the other ethylenically unsaturated compounds is within the above-described ranges, adhesiveness and peelability of a cured product become more excellent and production of the nail cosmetic material is facilitated.

### <Content of Monomer Component>

It is preferable that the nail cosmetic material of the present invention contains, with respect to 100 parts by mass of the nail cosmetic material, 1 to 25 parts by mass of the component 1 and 1 to 50 parts by mass of the component 2.

In addition, the nail cosmetic material of the present invention preferably contains 10 to 80 parts by mass of the component 1, the component 2, and the other ethylenically unsaturated compounds as a total amount with respect to 100 parts by mass of the nail cosmetic material, and more preferably contains 20 to 70 parts by mass thereof as a total amount with respect to 100 parts by mass of the nail cosmetic material.

### <Photopolymerization Initiator>

The nail cosmetic material of the present invention preferably contains a photopolymerization initiator and more preferably contains a photoradical polymerization initiator.

Any well-known photopolymerization initiator can be suitably used as the photopolymerization initiator. Although specific examples of the photopolymerization initiator will be shown below, the present invention is not limited thereto.

Acetophenone compounds (for example, 1-hydroxycyclohexyl phenyl ketone, 2,2-dimethoxy-2-phenylacetophenone, 2-hydroxy-2-methyl propiophenone, 4'-isopropyl-2-hydroxy-2-methyl propiophenone, 2-methyl-1-[4-(methylthio)phenyl]-2-morpholino-1-propane, 2-benzyl-2-dimethylamino-1-(4-morpholinophenyl)-butan-1-one, 1-[4-(2-hydroxyethoxy)phenyl]-2-hydroxy-2-methyl-1-propan-1-one, 1-[4-(2-(2-hydroxyethoxy)ethoxy)phenyl]-2-hydroxy-2-methyl-1-propan-1-one, benzoin, or benzoin methyl ether);
Benzophenone compounds (for example, 4,4'-bis(dimethylamino) benzophenone, or 3,3-dimethyl-4-methoxy-benzophenone);
Anthraquinone compounds (for example, anthraquinone, 2-methyl anthraquinone, 2-ethyl anthraquinone, or tert-butyl anthraquinone);
Thioxanthone compounds (for example, 2-chlorothioxanthone, diethyl thioxanthone, isopropyl thioxanthone, or diisopropyl thioxanthone);
Trihaloalkyl compounds (for example, 2,4,6-(trichloromethyl)triazine, 2,4-trichloromethyl-6-(4-methoxyphenyl)triazine, or tribromomethyl phenyl sulfone);
Lophine dimer compounds (for example, 2-(o-chlorophenyl)-4,5-diphenylimidazolyl dimer);
Acridine compounds (for example, 9-phenylacridine, 1,7-bis(9-acridinyl)heptane, 1,5-bis(9-acridinyl)pentane, or 1,3-bis(9-acridinyl)propane);
Phosphine oxide compounds (for example, trimethylbenzoyl diphenylphosphine oxide);
Metallocene compounds (for example, biscyclopentadienyl bis(difluoropyrrylphenyl)titanium); and
Onium salt compounds (for example, bis(4-t-butylphenyl)iodonium tosylate or triphenyl sulfonium tosylate).

The photopolymerization initiator can be appropriately selected according to the wavelength of light emitted by a light source used for exposure. However, at least one compound selected from the group consisting of an acetophenone compound, a benzophenone compound, a thioxanthone compound, a phosphine oxide compound, a metallocene compound, and a lophine dimer compound is preferably used, and at least one compound selected from the group consisting of acetophenone compound, a benzophenone compound, a thioxanthone compound, and a phosphine oxide compound is more preferably used.

In addition, the photopolymerization initiator can be appropriately selected according to the composition of the nail cosmetic material. Specifically, a photopolymerization initiator to be used is preferably selected so as to be dissolved in a specific compound described below, an ethylenically unsaturated compound other than the specific compound, and/or a solvent.

The photopolymerization initiator may be used singly or in combination of two or more thereof.

The content of the photopolymerization initiator in the nail cosmetic material of the present invention is, with respect to 100 parts by mass of the nail cosmetic material, preferably 0.1 to 20 parts by mass, more preferably 0.5 to 15 parts by mass, and particularly preferably 1 to 10 parts by mass. In a case where the content of the photopolymerization initiator is within the above-described ranges, the adhesiveness of a cured product becomes more excellent.

### <Polymer and/or Oligomer>

The nail cosmetic material of the present invention preferably contains a polymer and/or an oligomer.

The weight-average molecular weight of the polymer and/or the oligomer used in the present invention is preferably 750 to 300,000, more preferably 2,000 to 300,000, still more preferably 3,000 to 200,000, particularly preferably 4,000 to 150,000, and most preferably 5,000 to 100,000. In a case where the weight-average molecular weight of the polymer and/or the oligomer is within the above-described ranges, adhesiveness and temporal stability of an obtained cured product become excellent.

In the present invention, a compound having a weight-average molecular weight of greater than or equal to 750 and less than 5,000 is called an oligomer and a compound having a weight-average molecular weight of greater than or equal to 5,000 is called a polymer.

A method for measuring the weight-average molecular weight of the polymer and the oligomer in the present invention is performed through gel permeation chromatography (GPC) based on a polystyrene standard or a polyethylene oxide standard.

The structure of the polymer and/or the oligomer that can be used in the present invention is not particularly limited, and an arbitrary structure may be used. Examples thereof include a chain structure, a branched structure, a star structure, a crosslinking structure, and a network structure.

The types of polymers and/or oligomers are not particularly limited, and any well-known types of polymers (poly(meth)acrylic acid ester, poly(meth)acrylic acid amide, polyurethane, polyester, polyether, polyurea, polycarbonate, polyamide, polystyrene, polyalkylene, and polyvinyl) can be used.

The polymer and/or the oligomer preferably have a radically polymerizable ethylenically unsaturated group. A polymer and/or an oligomer having a (meth)acryloyl group are preferably used.

The polymer and/or the oligomer are preferably a polymer and/or an oligomer having a structure represented by Formula C-1, and are particularly preferably a polymer and/or an oligomer having the structure represented by Formula C-1 in a polymer main chain. In the case of the embodiment, the adhesiveness becomes particularly excellent.

The polymer and/or the oligomer are more preferably a polymer and/or an oligomer having a urethane bond, and are particularly preferably a polymer and/or an oligomer having a urethane bond in a polymer main chain. In the case of the embodiment, the adhesiveness becomes particularly excellent.

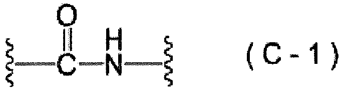

In Formula C-1, the wavy line portions each independently represent a bonding position with other structures.

Specific examples of the polymer and/or the oligomer used in the present invention are shown below, but the present invention is not limited thereto.

**[Table 1]**

| Number | Unit-1 | | Unit-2 | | Unit-3 | | Unit-4 | | Unit-5 | | Mw |
|---|---|---|---|---|---|---|---|---|---|---|---|
| P-16 | TDI | 50 | Diol-9 | 30 | Diol-5 | 18 | HEMA | 2 | - | - | 20,000 |
| P-17 | TDI | 50 | Diol-1 | 30 | Diol-2 | 18 | HPMA | 2 | - | - | 20,000 |
| P-18 | MDI | 40 | HDI | 10 | Diol-6 | 18 | Diol-10 | 30 | Methanol | 2 | 22,000 |
| P-19 | ADPA | 50 | Diamine-2 | 48 | n-butylamine | 2 | - | - | - | - | 30,000 |
| P-20 | MMA | 50 | BMA | 50 | - | - | - | - | - | - | 35,000 |
| P-21 | ADPA | 50 | Diol-8 | 48 | Methanol | 2 | - | - | - | - | 15,000 |
| P-22 | Nitrocellulose | | | | | | | | | | |

The numerical values denoted in the columns of Unit-1 to Unit-5 in Table 1, represent molar ratios of monomer units derived from compounds forming each unit.

The description "-" in the columns of Unit-3 to Unit-5 indicates that the corresponding unit is not included.

The details of the compounds forming each unit described in Table 1 are shown below.
TDI: tolylene diisocyanate
Diol-9: 2,2-bis(4-hydroxycyclohexyl)propane
Diol-5: polytetramethylene oxide (polytetramethylene ether glycol, Mw:2,000)
HEMA: 2-hydroxyethyl methacrylate
Diol-1: 3-dimethylamino-1,2-propanediol
Diol-2: polycarbonate diol composed of 1,5-penthanediol and 1,6-hexanediol (Mw: 2,000)
HPMA: 2-hydroxypropyl methacrylate
MDI: bis(4-isocyanatophenyl)methane
HDI: hexamethylene diisocyanate
Diol-6: polypropylene glycol (Mw: 1,000)
Diol-10: glycerin monomethacrylate
MMA: methyl methacrylate
BMA: n-butyl methacrylate
ADPA: adipic acid dichloride
Diol-8: 1,4-butanediol
Nitrocellulose: DHX40-70 (manufactured by Inabata & Co., Ltd)
Diamine-2: polypropylene glycol diamine (Mw: 2,000)

In addition, U-6LPA and U-15HA (all manufactured by SHIN-NAKAMURA CHEMICAL CO., LTD.) are also preferably exemplified as the polymer and/or the oligomer used in the present invention.

The polymer and/or the oligomer used in the present invention are preferably a polymer and/or an oligomer having an amino group.

Any of primary, secondary, and tertiary amino groups can be used as the amino groups. However, A tertiary amino group is preferable from the viewpoints of easiness of production of a polymer and/or an oligomer, adhesiveness and peelability of an obtained cured film, and temporal stability.

The introduction position of an amino group, particularly a tertiary amino group, in the polymer and/or the oligomer may be any of the side chain, the inside of a main chain, and a terminal of a main chain, and may be two or more positions.

A group represented by Formula C-2 or Formula C-3 is preferable as the tertiary amino group.

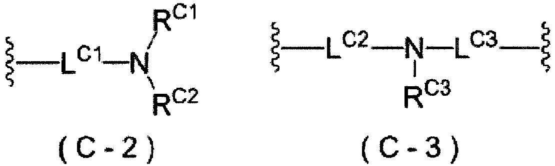

In Formula C-2, R^{C1} and R^{C2} each independently represent a hydrogen atom or an alkyl group having 1 to 10 carbon atoms, L^{C1} represents a divalent linking group; at least two selected from the group consisting of R^{C1}, R^{C2}, and L^{C1} may be linked to each other to form a ring; and the wavy line portions represent linking positions with other structures.

R^{C3} in Formula C-3 represents a hydrogen atom or an alkyl group having 1 to 10 carbon atoms; L^{C2} and L^{C3} each independently represent a divalent linking group; at least two selected from the group consisting of R^{C3}, L^{C2}, and L^{C3} may be linked to each other to form a ring; and the wavy line portions each independently represent linking positions with other structures.

R^{C1} and R^{C2} in Formula C-2 is preferably an alkyl group having 1 to 10 carbon atoms, more preferably an alkyl group having 1 to 6 carbon atoms, still more preferably an alkyl group having 1 to 4 carbon atoms, and particularly preferably an alkyl group having 1 or 2 carbon atoms from the viewpoint of a balance between solubility in an aqueous acid solution and water resistance.

Examples of L^{C1} in Formula C-2 include a single bond, an alkylene group having 1 to 20 carbon atoms (which may have a substituent or in which some carbon atoms may be substituted with hetero atoms), and an arylene group having 1 to 20 carbon atoms (which may have a substituent or in which some carbon atoms may be substituted with hetero atoms). L^{C1} in Formula C-2 is preferably a single bond, an alkylene group having 1 to 20 carbon atoms, an oxyalkylene group having 2 to 20 carbon atoms, or a polyoxyalkylene group having 2 to 20 carbon atoms, more preferably an alkylene group having 1 to 20 carbon atoms or a polyoxyalkylene group having 2 to 20 carbon atoms, particularly preferably an alkylene group having 1 to 20 carbon atoms, and most preferably an alkylene group having 1 to 10 carbon atoms.

In Formula C-2, at least two selected from the group consisting of R^{C1}, R^{C2}, and L^{C1} may be linked to each other to form a ring.

R^{C3} in Formula C-3 is preferably an alkyl group having 1 to 10 carbon atoms, more preferably an alkyl group having 1 to 6 carbon atoms, and still more preferably an alkyl group having 1 to 4 carbon atoms from the viewpoint of a balance between solubility in an aqueous acid solution and water resistance.

Examples of L^{C2} and L^{C3} in Formula C-3 each independently include a single bond, an alkylene group having 1 to 20 carbon atoms (which may have a substituent or in which some carbon atoms may be substituted with hetero atoms), and an arylene group having 1 to 20 carbon atoms (which may have a substituent or in which some carbon atoms may be substituted with hetero atoms). L^{C2} and L^{C3} in Formula C-3 are preferably a single bond, an alkylene group having 1 to 20 carbon atoms, an oxyalkylene group having 2 to 20 carbon atoms, or a polyoxyalkylene group having 2 to 20 carbon atoms, more preferably an alkylene group having 1 to 20 carbon atoms or a polyoxyalkylene group having 2 to 20 carbon atoms, particularly preferably an alkylene group having 1 to 20 carbon atoms, and most preferably an alkylene group having 1 to 10 carbon atoms.

In Formula C-3, at least two selected from the group consisting of R^{C3}, L^{C2}, and L^{C3} may be linked to each other to form a ring.

The amine value of the polymer and/or the oligomer used in the present invention is preferably 0.1 to 10 mmol/g, more preferably 0.25 to 9 mmol/g, and still more preferably 0.5 to 8 mmol/g. In a case where the amine value thereof is within the above-described ranges, the adhesiveness and the peelability become more excellent.

As a method for measuring the amine value, it is possible to obtain the amine value such that, for example, a sample is weighed in a beaker, acetic acid is added to the sample, the mixture is stirred and dissolved, the measurement temperature is adjusted to be 25°C, and then, titration is performed with a titration device using a 0.1 mol/L perchloric acid acetic acid solution as a titration reagent. The amine value is a value obtained by representing the amount of the perchloric acid consumed during the titration in terms of the number of moles per 1 g of the sample (solid content).

Specific examples of the polymer and/or the oligomer having an amino group used in the present invention will be shown below, but the present invention is not limited thereto.

**[Table 2]**

| Number | Unit-1 | | Unit-2 | | Unit-3 | | Unit-4 | | Unit-5 | | Mw |
|---|---|---|---|---|---|---|---|---|---|---|---|
| P-1 | IPDI | 50 | Diol-1 | 40 | Diol-2 | 8 | Methanol | 2 | - | - | 35,000 |
| P-2 | IPDI | 50 | Diol-3 | 40 | Diol-2 | 8 | Methanol | 2 | - | - | 34,000 |
| P-3 | IPDI | 50 | Diamine-1 | 39 | Diamine-2 | 9 | Morpholine | 2 | - | - | 13,000 |
| P-4 | ADPA | 50 | Diamine-1 | 39 | Diamine-2 | 9 | Morpholine | 2 | - | - | 22,000 |
| P-5 | ADPA | 50 | Diol-1 | 20 | Diol-4 | 28 | IPA | 2 | - | - | 35,000 |
| P-6 | DMAPAm | 50 | CHAm | 50 | - | - | - | - | - | - | 40,000 |
| P-7 | DMAEM | 50 | BMA | 50 | - | - | - | - | - | - | 45,000 |
| P-8 | IPDI | 50 | Diol-1 | 40 | Diol-2 | 8 | HEMA | 2 | - | - | 36,000 |
| P-9 | TDI | 50 | Diol-1 | 30 | Diol-5 | 18 | HEMA | 2 | - | - | 25,000 |
| P-10 | HDI | 50 | Diol-3 | 30 | Diol-5 | 18 | HEMA | 2 | - | - | 31,000 |
| P-11 | MDI | 50 | Diol-1 | 48 | Ethanol | 2 | - | - | - | - | 23,000 |
| P-12 | TDI | 50 | Diamine-1 | 39 | Diamine-2 | 9 | Piperidine | 2 | - | - | 10,000 |
| P-13 | ADPA | 50 | Diamine-2 | 48 | DMPA | 2 | - | - | - | - | 8,000 |
| P-14 | IPDI | 50 | Diamine-2 | 48 | DMPA | 2 | - | - | - | - | 9,000 |
| P-15 | MDI | 40 | HDI | 10 | Diol-6 | 10 | Diol-7 | 25 | Diol-8 | 15 | 73,000 |

The numerical values denoted in columns of each unit in Table 2 represent molar ratios of monomer units derived from compounds forming each unit.

The description "-" in the columns of Unit-3 to Unit-5 indicates that the corresponding unit is not included.

The details of the compounds forming each unit described in Table 2 are shown below.
IPDI: isophorone diisocyanate
TDI: tolylene diisocyanate
HDI: hexamethylene diisocyanate
MDI: bis(4-isocyanatophenyl)methane
Diol-1: 3-dimethylamino-1,2-propanediol
Diol-2: polycarbonate diol composed of 1,5-penthanediol and 1,6-hexanediol (Mw: 2,000)
Diol-3: N-butyl diethanolamine
Diol-4: 1,5-penthanediol
Diol-5: polytetrabutylene glycol (Mw: 2,000)
Diol-6: polypropylene glycol (Mw: 1,000)
Diol-7: 3-diethylamino-1,2-propanediol
Diol-8: 1,4-butanediol
Diamine-1: 3,3-diamine-N-methyl dipropylamine
Diamine-2: polypropylene glycol diamine (Mw: 2,000)
ADPA: adipic acid dichloride
DMAPAm: 3-dimethylaminopropyl acrylamide
CHAm: N-cyclohexyl acrylamide
DMAEM: 2-dimethylaminoethyl methacrylate
BMA: n-butyl methacrylate
HEMA: 2-hydroxyethyl methacrylate
IPA: 2-hydroxypropane
DMPA: 3-dimethylaminopropylamine

The polymer and/or the oligomer used in the present invention can be produced through a well-known method (for example, radical polymerization or polycondensation).

The polymer and/or the oligomer may be used singly or in combination of two or more thereof.

The content of the polymer and/or the oligomer in the nail cosmetic material used in the present invention is, with respect to 100 parts by mass of the nail cosmetic material, preferably greater than 0 parts by mass and less than or equal to 90 parts by mass, more preferably 5 to 80 parts by mass, and still more preferably 10 to 60 parts by mass. In a case where the content of the polymer and/or the oligomer is within the above-described ranges, the adhesiveness and the peelability of a cured product become more excellent.

A water-soluble polymer and/or an oligomer can also be suitably used as the polymer and/or the oligomer used in the present invention.

The water-soluble polymer and/or the oligomer is preferably a polymer and/or an oligomer that dissolves in 100 g of distilled water at 25°C in an amount of greater than or equal to 0.1 g, more preferably a polymer and/or an oligomer that dissolves in 100 g of distilled water at 25°C in an amount of greater than or equal to 0.2 g, and particularly preferably a polymer and/or an oligomer that dissolves in 100 g of distilled water at 25°C in an amount of greater than or equal to 0.5 g. In the case where the polymer and/or the oligomer have water solubility within the above-described ranges, production of a nail cosmetic material using water becomes facilitated.

A polymer and/or an oligomer having a functional group selected from the group consisting of a carboxylic acid (salt) group, a sulfonic acid (salt) group, a phosphoric acid (salt) group, a phosphonic acid (salt) group, a quaternary ammonium salt group, a hydroxyl Group, a carboxylic acid amide group, and a polyethylene glycol chain are preferable as the water-soluble polymer and/or the oligomer used in the present invention.

As counter cations of a carboxylic acid (salt) group, a sulfonic acid (salt) group, a phosphoric acid (salt) group, and a phosphonic acid (salt) group, alkali metal cations such as sodium and potassium, alkaline earth metal cations such as calcium and magnesium, an ammonium cation, and a phosphonium cation are preferable, and alkali metal cations such as sodium and potassium are particularly preferable.

A methyl group or an ethyl group is preferable as an alkyl group of an ammonium group of a quaternary ammonium salt group. In addition, as counter anions, halide ions such as a chloride ion and a bromide ion, a sulfate anion, a nitrate anion, a phosphate anion, a sulfonate anion, a carboxylate anion, and a carbonate anion are preferable, and a halide ion, a sulfonate anion, and a carboxylate anion are particularly preferable.

As a substituent on nitrogen of a carboxylic acid amide group, an alkyl group having less than or equal to 8 carbon atoms is preferable, and an alkyl group having less than or equal to 6 carbon atoms is particularly preferable.

The linking chain length of polyethylene glycol chains is preferably greater than or equal to 2 and particularly preferably greater than or equal to 4.

Specific examples of the water-soluble polymer and/or the oligomer used in the present invention will be shown below, but the present invention is not limited thereto.

**[Table 3]**

| Number | Unit-1 | | Unit-2 | | Unit-3 | | Unit-4 | | Unit-5 | | Mw |
|---|---|---|---|---|---|---|---|---|---|---|---|
| P-23 | MDI | 50 | Diol-6 | 5 | Diol-11 | 25 | Diol-10 | 18 | Methanol | 2 | 100,000 |
| P-24 | TDI | 50 | Diol-12 | 18 | Diol-9 | 30 | HEMA | 2 | - | - | 20,000 |
| P-25 | Polymer represented by Formula C' | | | | | | | | | | 20,000 |

The numerical values denoted in columns of each unit in Table 3 represent molar ratios of monomer units derived from compounds forming each unit.

The description "-" in the column of Unit-5 indicates that the corresponding unit is not included.

The details of the compounds forming each unit described in Table 3 are shown below.
MDI: bis(4-isocyanatophenyl)methane
Diol-6: polypropylene glycol (Mw: 1,000)
Diol-11: sodium 2,2-bis(hydroxymethyl) propionate
Diol-10: glycerin monomethacrylate
TDI: tolylene diisocyanate
Diol-12: polyethylene glycol (Mw: 2,000)
Diol-9: 2,2-bis(4-hydroxycyclomethyl)propane
HEMA: 2-hydroxyethyl methacrylate

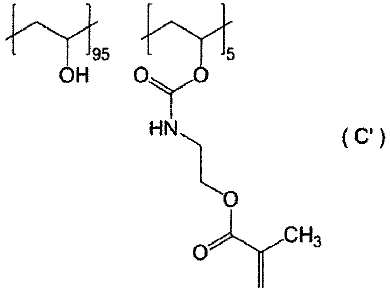

The figures on the lower right sides of the parentheses in the polymer represented by Formula C' represent molar ratios.

The polymer and/or the oligomer used in the present invention can be produced through a well-known method (for example, radical polymerization or polycondensation).

The polymer and/or the oligomer may be used singly or in combination of two or more thereof.

The content of the polymer and/or the oligomer in the nail cosmetic material used in the present invention is, with respect to 100 parts by mass of the nail cosmetic material, preferably greater than 0 parts by mass and less than or equal to 90 parts by mass, more preferably 5 to 80 parts by mass, and still more preferably 10 to 60 parts by mass. In a case where the content of the polymer and/or the oligomer is within the above-described ranges, the adhesiveness and the peelability of a cured product become more excellent.

### <Solvent>

The nail cosmetic material of the present invention may contain a solvent in order to improve the coatability of the nail cosmetic material.

As the solvent, a solvent having a boiling point of 50°C to 150°C at 1 atmosphere (1013.25 hPa) is preferable, and a solvent having a boiling point of 60°C to 140°C at 1 atmosphere is more preferable. In a case where a solvent having a boiling point within the above-described ranges, concentration fluctuation of the nail cosmetic material can be suppressed and drying after coating is favorably performed.

Examples of the solvent include: water; alcohols such as methanol, ethanol, isopropyl alcohol, 1-propanol, 1-butanol, 2-butanol, t-butyl alcohol, 2-methyl-1-propanol, and 1-methoxy-2-propyl alcohol; hydrocarbons such as hexane, heptane, toluene, and xylene; ethers such as diethyl ether, diisopropyl ether, tetrahydrofuran, dimethoxyethane, and diphenyl ether; esters such as methyl acetate, ethyl acetate, butyl acetate, and γ-butyrolactone; ketones such as acetone, methyl ethyl ketone, and cyclohexanone; amides such as N,N-dimethylformamide, N,N-dimethylacetamide, N-methylpyrrolidone, and N-ethylpyrrolidone; carbonates such as dimethyl carbonate, diethyl carbonate, and propylene carbonate; ureas such as tetramethyl urea and 1,3-dimethyl-2-imidazolidinone; halogenated hydrocarbons such as chloroform, dichloromethane, and chlorobenzene; and well-known solvents such as triethylamine, acetic acid, acetonitrile, nitromethane, and dimethyl sulfoxide.

Among these solvents, water, methanol, ethanol, isopropyl alcohol, 1-propanol, 1-butanol, 2-butanol, t-butyl alcohol, 2-methyl-1-propanol, and 1-methoxy-2-propyl alcohol, acetic acid, and/or acetonitrile are preferable, and water, ethanol, isopropyl alcohol, and/or acetic acid are more preferable. By using the solvents, it is possible to reduce the viscosity derived from the component 1 of the present invention and to improve the coatability.

The solvent may be contained singly or in combination of two or more thereof.

In a case where the nail cosmetic material of the present invention contains the solvents, the content of the solvent in the nail cosmetic material of the present invention is, with respect to 100 parts by mass of the nail cosmetic material, preferably greater than 1 part by mass and less than or equal to 60 parts by mass, more preferably 5 to 50 parts by mass, and still more preferably 10 to 40 parts by mass. In a case where the content of the solvent is within the above-described ranges, the coatability and the drying properties of the nail cosmetic material become favorable.

In addition, regarding the mixing ratio of the component 1 to the solvent, the component 1 is, with respect to 100 parts by mass of the solvent, preferably 5 to 200 parts by mass, more preferably 7 to 150 parts by mass, and still more preferably 10 to 100 parts by mass. In a case where the mixing ratio thereof is within the above-described ranges, the production of the nail cosmetic material becomes facilitated.

### <Other Components>

In addition to the above-described components, any well-known compounds can be added to the nail cosmetic material of the present invention as necessary. Specific examples thereof include additives such as a sensitizer, a polymerization inhibitor, a pigment, a dye, a perfume, an ultraviolet (UV) absorbent, an antioxidant, a filler, various elastomers, a plasticizer, a thickener, a thixotropy imparting agent, a silane coupling agent, a titanate coupling agent, a chelating agent, a flame retardant, and a surfactant.

### <Method for Applying Nail Cosmetic Material>

Examples of the method for applying a nail cosmetic material using the nail cosmetic material of the present invention preferably include a method for coating an object such as a nail, a nail on which other nail cosmetic materials are applied, or an artificial nail with the nail cosmetic material of the present invention to form a cured product while irradiating the nail cosmetic material with light.

The method for coating an object with the nail cosmetic material of the present invention is not particularly limited, and may be performed through well-known methods. Examples thereof include a method for coating the nail cosmetic material using a paint brush, an ink brush, or the like, spray coating, inkjet coating, bar coater coating, rotary coating, curtain coating, dip coating, air knife coating, blade coating, and roll coating.

The coating thickness of the nail cosmetic material of the present invention on an object varies in accordance with its use. The film thickness after curing is preferably 1 nm to 1 mm, more preferably 10 nm to 0.5 mm, and still more preferably 100 nm to 0.25 mm. In a case where the film thickness is within the above-described ranges, more favorable adhesiveness and peelability of a cured product is achieved.

The method for coating an object with the nail cosmetic material of the present invention and irradiating the nail cosmetic material with light is not particularly limited. Any well-known light irradiation method (for example, whole surface exposure or scanning exposure) can be used in which a well-known light source (for example, solar light, a high pressure mercury lamp, a fluorescent lamp, a UV lamp, a light emitting diode (LED) lamp, or an LED laser) is used. The light irradiation time is not particularly limited as long as the nail cosmetic material of the present invention is cured.

As the method for forming a cured product of the nail cosmetic material on an object, the above-described method can be repeatedly performed.

As a method for removing a cured film obtained by curing the nail cosmetic material of the present invention, the nail cosmetic material can be removed through well-known methods. Specific examples thereof include a polishing method using a file or the like; a scraping method using a sculpture knife, a knife, or the like; a method for covering a cured film with cotton moistened with acetone and allowing the cured film to stand for a few minutes to make the cured film brittle, and then, peeling or crushing the cured film with a bar or the like; a method for immersing a cured film in an aqueous solution having a specific pH for a few minutes to make the cured film brittle, and then, peeling the cured film with tweezers or the like or pushing and scraping the cured film with a bar; and a method in which these methods are combined.

Particularly, preferred examples of the method for removing a cured film obtained by curing the nail cosmetic material of the present invention from a nail include method for covering a cured film with cotton moistened with acetone and allowing the cured film to stand for a few minutes to make the cured film brittle, and then, peeling or crushing the cured film with a bar or the like; a method for immersing a cured film in an aqueous solution having a specific pH for a few minutes to make the cured film brittle, and then, peeling the cured film with tweezers or the like or pushing and scraping the cured film with a bar; and a method in which these methods are combined. More preferred example of the method for removing a cured film obtained by curing the nail cosmetic material of the present invention from a nail include a method for immersing a cured film in an aqueous solution having a specific pH for a few minutes to make the cured film brittle, and then, peeling the cured film with tweezers or the like or pushing and scraping the cured film with a bar.

### (Nail Art Kit)

The nail art kit of the present invention includes the nail cosmetic material of the present invention and one or more tools for applying the nail cosmetic material on a nail.

A preferred embodiment of the nail cosmetic material of the present invention in the nail art kit of the present invention is as described above.

Specific examples of the tools for applying a nail cosmetic material on a nail include a nail cosmetic material other than the nail cosmetic material of the present invention for color layer, a top layer, or the like; a nail file such as a file; an ink brush such as a flat brush, or a paint brush, for applying a nail cosmetic material; a device of exposing UV light or the like; a wiping or cleaning liquid; a wipe for wiping; a nail brush; a dust brush; a nail form used for repair of a nail, decorative stones made of acryl, glass, metal, natural stone, or the like; nail seal; decorative powder such as glitter or hologram; a cutter; a spatula; a stick; tweezers; and a separator for separating the distances between fingers in order to prevent contact between nails. However, the present invention is not limited thereto.

### [Examples]

Hereinafter, the present invention will be more specifically described using examples, but the present invention is not limited to embodiments in these examples. Unless otherwise specified, "parts" and "%" are on a mass basis.

In addition, polymers P-16 and P-17 used in the examples are respectively the same as those described above.

### (Examples 1 to 12 and Comparative Examples 1 to 8)

### 1. Preparation of Nail Cosmetic Material

In Examples 1 to 12 and Comparative Examples 1 to 8, monomers described in Tables 4 to 7 were placed in a container provided with a stirring device. Then, the monomers were heated to 55°C using a heating and stirring device, and were mixed with each other until the monomers became homogeneous to prepare a monomer mixed liquid. Next, each polymer described in Tables 4 to 7 and a photoinitiator were weighed in a separate container, and a necessary amount of the monomer mixed liquid was weighed and mixed therewith to obtain nail cosmetic materials of the present invention.

The numerical values denoted in columns of Component 1, Component 2, and Other ethylenically unsaturated compounds, Polymer, and Photopolymerization initiator in Tables 4 to 7 indicate the amount (parts by mass) of each monomer added, and the columns with "-" indicate that the corresponding compound is not contained.

### 2. Evaluation of Coatability

In Examples 1 to 12 and Comparative Examples 1 to 8, nails of the hand were coated with the obtained nail cosmetic materials using a paint brush, and the coatability (stringiness or self-leveling properties) was evaluated according to the following evaluation criteria. The evaluation results are described in Table 8.

### -Evaluation Criteria-

A: There is no stringiness, and smoothing of the coated surface proceeds sufficiently after 10 seconds.
B: There is slight stringiness, and the coated surface has not been smoothed sufficiently even after 10 seconds.
C: There is stringiness, and the coated surface is not smoothed even after 10 seconds.

### 3. Formation of Base Coat Layer (Base Layer)

After the nails of the hand were coated with the nail cosmetic materials in Examples 1 to 12 and Comparative Examples 1 to 8 using a paint brush, the nail cosmetic materials were photocured (exposed for 60 seconds) using a gel nail exclusive UV device (36 W) to form base coat layers. The thickness of each base coat layer formed is about 100 µm.

### 4. Formation of Gel Nail Layer

Next, each obtained base coat layer was coated with commercially available CALGEL #CG-03 FRESH PINK (manufactured by MOGA BROOK Co., ltd.) using a paint brush, and then, irradiation was performed for 1 minute using an ultraviolet lamp (36 W) which is a gel nail exclusive UV device. Thereafter, the base coat layer was coated with commercially available TOP GEL (manufactured by MOGA BROOK Co., ltd.) as a top layer using a paint brush, and irradiation was similarly performed for 2 minutes using the ultraviolet lamp (36W). Each gel nail layer formed was visually observed, and as a result, the gel nail layer was completely cured. The thickness of each film in which the color layer and the top layer were combined was about 250 µm.

### [Evaluation of Adhesiveness]

### (1) External Force Resistance

The adhesiveness of each nail/base coat layer/gel nail layer laminate obtained in Examples 1 to 12 and Comparative Examples 1 to 8 was evaluated from the viewpoint of external force resistance. That is, a distal portion of each nail including a base coat layer and a gel nail layer was rubbed with the top of a commercially available rubber sheet 30 times, and then, the change in the distal portion was evaluated according to the following criteria. The evaluation results are shown in Table 8. In all examples and comparative examples, peeling between each base coat layer and each gel nail layer was not checked.

In Table 8, a total value of evaluation values of adhesiveness evaluated from the viewpoint of external force resistance, adhesiveness evaluated from the viewpoint of warm water resistance, and peelability is described in the column denoted as "Total value of evaluation values".

### -Evaluation Criteria-

4: No change in appearance was checked.
3: Slight peeling was checked between a nail and a base coat layer.
2: Partial peeling was checked between a nail and a base coat layer, and whitening was checked in a part of the appearance.
1: Significant peeling was checked between a nail and a base coat layer, and whitening was checked on the whole appearance.

### [Evaluation of Peelability]

The peelability of each nail/base coat layer/gel nail layer laminate obtained in Examples 1 to 12 and Comparative Examples 1 to 8 was evaluated. That is, cloth containing acetone was wound on each nail on which a base coat layer and a gel nail layer were formed, and the nail was allowed to stand for 15 minutes. Then, the laminate was lightly scrubbed with a commercially available orange stick to check whether or not it is possible to peel the laminate. In a case where it was impossible to peel the laminate in one cycle of this operation, the same cycle was repeatedly performed, and the peelability was evaluated using the number of cycles, required for the laminate to be peeled, according to the following criteria. The obtained results are shown in Table 8.

### -Evaluation Criteria-

4: A laminate of a base coat layer and a gel nail layer was peeled from a nail in one cycle.
3: A laminate of a base coat layer and a gel nail layer was peeled from a nail in two cycles.
2: A laminate of a base coat layer and a gel nail layer was peeled from a nail in three cycles.
1: A laminate of a base coat layer and a gel nail layer was not peeled from a nail even in three cycles.

**[Table 4]**

| | Component 1 | Component 2 | Other ethylenically unsaturated compounds | | | | Total amount of monomers | Polymer | Total amount of monomers and polymer | Photopolymerization initiator | |
|---|---|---|---|---|---|---|---|---|---|---|---|
| | HEAA | DMAA | HEMA | IBXMA | IBXA | NVP | | P-16 | | TPO | Irg 184 |
| Example 1 | 20 | 10 | - | 20 | - | - | 50 | 50 | 100 | 4.0 | 4.0 |
| Example 2 | 15 | 15 | - | 20 | - | - | 50 | 50 | 100 | 4.0 | 4 0 |
| Example 3 | 10 | 20 | - | 20 | - | - | 50 | 50 | 100 | 4.0 | 40 |
| Comparative Example 1 | 15 | - | 15 | - | 20 | - | 50 | 50 | 100 | 4.0 | 4.0 |
| Comparative Example 2 | 15 | - | - | - | 20 | 15 | 50 | 50 | 100 | 4.0 | 4.0 |

**[Table 5]**

| | Component 1 | Component 2 | Other ethylenically unsaturated compounds | | Total amount of Monomers | Polymer | Total amount of monomers and polymer | Photopolymerization initiator |
|---|---|---|---|---|---|---|---|---|
| | HEAA | DEAA | HEMA | NVP | | P-17 | | TPO |
| Example 4 | 20 | 10 | 10 | - | 40 | 60 | 100 | 4.0 |
| Example 5 | 15 | 15 | 10 | - | 40 | 60 | 100 | 4.0 |
| Example 6 | 10 | 20 | 10 | - | 40 | 60 | 100 | 4.0 |
| Comparative Example 3 | 15 | - | 25 | - | 40 | 60 | 100 | 4.0 |
| Comparative Example 4 | 15 | - | - | 25 | 40 | 60 | 100 | 4.0 |

**[Table 6]**

| | Component 1 | Component 2 | Other ethylenically unsaturated compounds | | Total amount of Monomers | Polymer | Total amount of monomers and polymer | Photopolymerization initiator | |
|---|---|---|---|---|---|---|---|---|---|
| | HEAA | ACMO | IBXA | Acrylic acid | | P-16 | | TPO | Irg 184 |
| Example 7 | 20 | 10 | 15 | 5 | 50 | 50 | 100 | 3.0 | 1.0 |
| Example 8 | 15 | 15 | 15 | 5 | 50 | 50 | 100 | 3.0 | 1.0 |
| Example 9 | 10 | 20 | 15 | 5 | 50 | 50 | 100 | 3.0 | 1.0 |
| Comparative Example 5 | 15 | - | 30 | 5 | 50 | 50 | 100 | 3.0 | 1.0 |
| Comparative Example 6 | 15 | - | 15 | 20 | 50 | 50 | 100 | 3.0 | 1,0 |

**[Table 7]**

| | Component 1 | Component 2 | Other ethylenically unsaturated compounds | | Total amount of Monomers | Polymer | Total amount of monomers and polymer | Photopolymerization initiator | |
|---|---|---|---|---|---|---|---|---|---|
| | HEAA | LIGHT ESTER | HEMA | IBXMA | | P-17 | | TPO | Irg 184 |
| Example 10 | 20 | 10 | - | - | 30 | 70 | 100 | 4.0 | 4.0 |
| Example 11 | 15 | 15 | - | - | 30 | 70 | 100 | 4.0 | 4.0 |
| Example 12 | 10 | 20 | - | - | 30 | 70 | 100 | 4.0 | 4.0 |
| Comparative Example 7 | 15 | - | - | 15 | 30 | 70 | 100 | 4.0 | 4.0 |
| Comparative Example 8 | 15 | - | 15 | - | 30 | 70 | 100 | 4.0 | 4.0 |

**[Table 8]**

| Table 8 | Coatability | External force resistance | Peelability |
|---|---|---|---|
| Example 1 | A | 4 | 4 |
| Example 2 | A | 4 | 4 |
| Example 3 | A | 3 | 3 |
| Comparative Example 1 | B | 3 | 3 |
| Comparative Example 2 | C | 3 | 2 |
| Example 4 | A | 4 | 3 |
| Example 5 | A | 4 | 3 |
| Example 6 | A | 3 | 3 |
| Comparative Example 3 | B | 3 | 1 |
| Comparative Example 4 | C | 2 | 2 |
| Example 7 | A | 4 | 3 |
| Example 8 | A | 4 | 4 |
| Example 9 | A | 3 | 4 |
| Comparative Example 5 | C | 3 | 2 |
| Comparative Example 6 | B | 3 | 2 |
| Example 10 | A | 4 | 3 |
| Example 11 | A | 4 | 4 |
| CExample 12 | A | 3 | 2 |
| Comparative Example 7 | C | 3 | 3 |
| Comparative Example 8 | B | 3 | 2 |

The details of the compounds and the abbreviations denoted in Tables 4 to 7 are as follows.
HEAA: hydroxyethyl acrylamide (manufactured by KJ Chemicals Corporation)
DMAA: dimethyl acrylamide (manufactured by KJ Chemicals Corporation)
DEAA: diethyl acrylamide (manufactured by KJ Chemicals Corporation)
ACMO: acryloyl morpholine (manufactured by KJ Chemicals Corporation)
LIGHT ESTER BC: butoxy diethylene glycol methacrylate (manufactured by KYOEISHA CHEMICAL Co., LTD)
HEMA: 2-hydroxyethyl methacrylate (manufactured by KYOEISHA CHEMICAL Co., LTD)
IBXMA: isobornyl methacrylate (manufactured by KYOEISHA CHEMICAL Co., LTD)
IBXA: isobornyl acrylate (manufactured by KYOEISHA CHEMICAL Co., LTD)
NVP: N-vinyl-2-pyrrolidone (manufactured by NIPPON SHOKUBAI CO., LTD.)
Acrylic acid: acrylic acid (manufactured by NIPPON SHOKUBAI CO., LTD.)
P-16: Above-described polymer (synthetic product)
P-17: Above-described polymer (synthetic product)
TPO: diphenyl(2,4,6-trimethylbenzoyl)phosphine oxide (photopolymerization initiator manufactured by BASF, LUCIRIN TPO)
Irg 184: 1-hydroxycyclohexyl phenyl ketone (photopolymerization initiator manufactured by BASF)

### <Synthesis Example 1>

111.90 parts of polytetramethylene oxide (manufactured by Wako Pure Chemical Industries, Ltd., average molecular weight of 2,000), 9.6 parts of 2,2'-bis(4-hydroxycyclohexyl)propane (also called hydrogenated bisphenol A) (manufactured by Tokyo Chemical Industry Co., Ltd.), and 349.85 parts of tetrahydrofuran (manufactured by Wako Pure Chemical Industries, Ltd.) were placed in a three-neck flask and stirred for I hour at 25°C. Next, 18.1 parts of tolylene diisocyanate (manufactured by Tokyo Chemical Industry Co., Ltd.) was added to the three-neck flask, 0.15 parts of NEOSTAN U-600 (inorganic bismuth catalyst manufactured by NITTO KASEI CO., LTD.) was further added to the three-neck flask, and the mixture was stirred for 5 hours at 40°C. Next, 10.4 parts of 2-hydroxyethyl methacrylate (manufactured by Wako Pure Chemical Industries, Ltd.) was added to the three-neck flask, and the mixture was stirred for 1 hour at 40°C. The mixture was cooled to 25°C, and a polymer reaction liquid was obtained. Precipitates were taken out and dried to obtain a polymer P-16.

The weight-average molecular weight (in terms of polystyrene) of the whole polymer P-16 which was measured through a GPC method was 20,000.

Synthesis was similarly performed on P-17 while changing the monomer to be used as shown in Table 1.

### (Examples 13 to 21 and Comparative Examples 9 to 15)

### 1. Preparation of Nail Cosmetic Material

Nail cosmetic materials in Examples 13 to 21 and Comparative Examples 9 to 15 were obtained similarly to Examples 1 to 12 and Comparative Examples 1 to 8 except that the types and the addition amounts of monomers to be used were changed as shown in Table 9.

The numerical values denoted in columns of Component 1, Component 2, and Other ethylenically unsaturated compounds, Polymer, and Photopolymerization initiator in Table 9 indicate the content (parts by mass) of each component in the nail cosmetic materials.

### 2. Evaluation of Coatability

The coatability of the nail cosmetic materials obtained in Examples 13 to 21 and Comparative Examples 9 to 15 was evaluated similarly to Examples 1 to 12 and Comparative Examples 1 to 8.

The evaluation results are described in Table 10.

### 3. Formation of Base Coat Layer (Base Layer)

After the nails of the hand were coated with the nail cosmetic materials in Examples 13 to 21 and Comparative Examples 9 to 15 using a paint brush, the nail cosmetic materials were photocured (exposed for 60 seconds) using a gel nail exclusive UV device (36 W) to form base coat layers. The thickness of each base coat layer formed is about 120 µm.

### 4. Formation of Gel Nail Layer

Next, each obtained base coat layer was coated with commercially available CALGEL #CG-03 FRESH PINK (manufactured by MOGA BROOK CO., ltd.) as a color layer using a paint brush, and then, irradiation was performed for 1 minute using an ultraviolet lamp (36 W). Thereafter, the base coat layer was coated with commercially available BIO GEL (manufactured by TAKARA BELMONT Corp.) as a top layer using a paint brush, and irradiation was similarly performed for 2 minutes using the ultraviolet lamp (36W). Each gel nail layer formed was visually observed, and as a result, the gel nail layer was completely cured. The thickness of each film in which the color layer and the top layer were combined was about 250 µm.

### [Evaluation of Adhesiveness]

The adhesiveness of each nail/base coat layer/gel nail layer laminate obtained in Examples 13 to 21 and Comparative Examples 9 to 15 was evaluated similarly to Examples 1 to 12 and Comparative Examples 1 to 8. The evaluation results are shown in Table 10. In all examples and comparative examples, peeling between each base coat layer and each gel nail layer was not checked.

### [Evaluation of Peelability]

The peelability of each nail/base coat layer/gel nail layer laminate obtained in Examples 13 to 21 and Comparative Examples 9 to 15 was evaluated similarly to Examples 1 to 12 and Comparative Examples 1 to 8. The obtained results are shown in Table 10.

**[Table 9]**

| | Component 1 | Components 2 | | | | Other ethylenically unsaturated compounds | | | | | Total amount of monomers | Polymers | | Oligomers | | Total amount of monomers polymers | Photopolymerization initiator | |
|---|---|---|---|---|---|---|---|---|---|---|---|---|---|---|---|---|---|---|
| | HEAA | DMAA | DEAA | ACMO | LIGHT ESTER | HEMA | IBXMA | IBXA | NVP | Acrylic acid | | P-16 | P-17 | U-6 LPA | U-15 HA | | TPO | Irg 184 |
| Example 13 | 15 | 10 | 10 | - | - | 10 | - | - | - | - | 45 | - | 55 | - | - | 100 | 4.0 | - |
| Example 14 | 15 | 10 | - | 10 | - | 10 | - | - | - | - | 45 | - | 55 | - | - | 100 | 4.0 | - |
| Example 15 | 15 | 10 | - | - | 10 | 10 | - | - | - | - | 45 | - | 55 | - | - | 100 | 4.0 | - |
| Example 16 | 15 | - | - | 10 | 10 | 10 | - | - | - | - | 45 | - | 55 | - | - | 100 | 4.0 | - |
| Example 17 | 10 | 20 | - | - | - | 10 | - | 20 | - | - | 60 | 40 | - | - | - | 100 | 4.2 | - |
| Example 18 | 30 | 10 | - | - | - | - | - | 20 | - | - | 60 | 40 | - | - | - | 100 | 4.2 | - |
| Example 19 | 20 | 20 | 10 | - | - | - | - | 40 | - | - | 90 | 10 | - | - | - | 100 | 8.0 | - |
| Example 20 | 15 | 10 | - | 10 | - | - | - | 20 | - | - | 55 | - | - | 45 | - | 100 | 5.0 | - |
| Example 21 | 15 | - | - | - | 20 | - | - | 20 | - | - | 55 | - | - | - | 45 | 100 | 3.5 | 3.5 |
| Comparative Example 9 | - | - | - | - | - | 40 | - | 20 | - | - | 60 | 40 | - | - | - | 100 | 4.2 | - |
| Comparative Example 10 | - | - | - | - | - | 30 | - | 20 | - | 10 | 60 | 40 | - | - | - | 100 | 4.2 | - |
| Comparative Example 11 | - | - | - | - | - | 20 | - | 20 | 10 | 10 | 60 | 40 | - | - | - | 100 | 4.2 | - |
| Comparative Example 12 | 15 | - | - | - | - | 10 | 35 | - | - | - | 60 | 40 | - | - | - | 100 | 4.2 | - |
| Comparative Example 13 | 10 | - | - | - | - | 20 | 30 | - | - | - | 60 | 40 | - | - | - | 100 | 42 | - |
| Comparative Example 14 | - | 20 | 20 | - | - | - | - | - | - | - | 40 | 60 | - | - | - | 100 | 4.0 | - |
| Comparative Example 15 | - | 20 | - | - | 20 | - | - | - | - | - | 40 | 60 | - | - | - | 100 | 4.0 | - |

**[Table 10]**

| | Coatability | Adhesiveness | Peelability |
|---|---|---|---|
| Example 13 | A | 4 | 3 |
| Example 14 | A | 4 | 4 |
| Example 15 | A | 4 | 3 |
| Example 16 | A | 4 | 3 |
| Example 17 | A | 4 | 3 |
| Example 18 | B | 4 | 4 |
| Example 19 | A | 3 | 3 |
| Example 20 | A | 3 | 4 |
| Example 21 | A | 4 | 3 |
| Comparative Example 9 | A | 2 | 2 |
| Comparative Example 10 | A | 2 | 2 |
| Comparative Example 11 | A | 1 | 3 |
| Comparative Example 12 | c | 3 | 3 |
| Comparative Example 13 | B | 3 | 3 |
| Comparative Example 14 | A | 2 | 2 |
| Comparative Example 15 | A | 1 | 3 |

### (Example 22 and Comparative Example 16)

### 1. Preparation of Nail Cosmetic Material

Monomer mixed liquids in Example 22 and Comparative Example 16 were prepared similarly to Examples 1 to 12 and Comparative Examples 1 to 8 except that the types and the addition amounts of monomers to be used were changed as described below.

Next, each of the polymer and the photopolymerization initiator described below and each monomer mixed liquid described above were weighed in a separate container in amounts described below and were mixed with each other to obtained nail cosmetic materials in Example 22 and Comparative Example 16.

### <Example 22>

### [Monomer]

Hydroxyethyl acrylamide (manufactured by KJ Chemicals Corporation): 10 parts by mass
Dimethyl acrylamide (manufactured by KJ Chemicals Corporation): 20 parts by mass
2-Hydroxyethyl methacrylate (manufactured by KYOEISHA CHEMICAL Co., LTD): 10 parts by mass
Isobornyl acrylate (manufactured by KYOEISHA CHEMICAL Co., LTD): 20 parts by mass

### [Polymer]

Urethane methacrylate polymer (P-16) (synthetic product): 40 parts by mass [Photopolymerization Initiator]
Diphenyl(2,4,6-trimethylbenzoyl)phosphine oxide (photopolymerization initiator manufactured by BASF, LUCIRIN TPO): 4.0 parts by mass

### [Solvent]

2-Propanol: 10 parts by mass

### <Comparative Example 16>

### [Monomer]

Hydroxyethyl acrylamide (manufactured by KJ Chemicals Corporation): 15 parts by mass
2-Hydroxyethyl methacrylate (manufactured by KYOEISHA CHEMICAL Co., LTD): 10 parts by mass
Isobornyl methacrylate (manufactured by KYOEISHA CHEMICAL Co., LTD): 35 parts by mass

### [Polymer]

Urethane methacrylate polymer (P-16) (synthetic product): 40 parts by mass [Photopolymerization Initiator]
Diphenyl(2,4,6-trimethylbenzoyl)phosphine oxide (photopolymerization initiator manufactured by BASF, LUCIRIN TPO): 4.2 parts by mass

### [Solvent]

2-Propanol: 10 parts by mass

### 2. Evaluation of Coatability

The coatability of the nail cosmetic materials obtained in Example 22 and Comparative Example 16 was evaluated similarly to Examples 1 to 12 and Comparative Examples 1 to 8.

The evaluation results are described in Table 11.

### 3. Formation of Base Coat Layer (Base Layer)

A nail-shaped aluminum substrate was coated with 0.20 g of a nail cosmetic material obtained in Example 22 or Comparative Example 16 using a paint brush, and was irradiated with an ultraviolet lamp (36W) for 2 minutes. In this manner, an aluminum substrate on which a base coat layer was formed was produced.

After washing the surface of the base coat layer with ethanol in order to measure the thickness of the cured film, the formed film was visually observed. As a result, the formed film had been completely cured. In addition, the film thickness at this time was about 80 µm (± 10 µm).

### 4. Formation of Gel Nail Layer

The aluminum substrate on which the base coat layer was formed was coated with commercially available CALGEL #CG-03 FRESH PINK (manufactured by MOGA BROOK CO., ltd.) as a color layer using a paint brush, and then, irradiation was performed for 1 minute using an ultraviolet lamp (36 W). Thereafter, the base coat layer was coated with commercially available TOP GEL (manufactured by MOGA BROOK CO., ltd.) as a top layer using a paint brush, and irradiation was similarly performed for 2 minutes using the ultraviolet lamp (36W). In this manner, an aluminum substrate 1, provided with a gel nail layer, and a base coat layer made of a cured product of the nail cosmetic material obtained in Example 22, was prepared. The gel nail layer formed was visually observed, and as a result, the gel nail layer was completely cured. The thickness of the film in which the color layer and the top layer were combined was about 250 µm.

An aluminum substrate 2 was produced similarly to Example 22 except that a cured product of the nail cosmetic material in Comparative Example 16 was used in a base coat layer in the same manner.

### [Evaluation of Adhesiveness and Peelability]

The adhesiveness and the peelability of the obtained aluminum substrate 1 and aluminum substrate 2 was evaluated through the same method as that in Examples 1 to 12 and Comparative Examples 1 to 8. The results are shown in Table 11. Regarding the evaluation of the adhesiveness, peeling between each base coat layer and each gel nail layer was not checked in any of Example 22 and Comparative Example 16.

**[Table 11]**

| | Coatability | Adhesiveness | Peelability |
|---|---|---|---|
| Example 22 | A | 3 | 3 |
| Comparative Example 16 | B | 2 | 2 |

## Claims

1. A nail cosmetic material comprising:
hydroxyethyl acrylamide as a component 1; and
at least one compound selected from the group consisting of dimethyl acrylamide, diethyl acrylamide, acryloyl morpholine, and butoxy diethylene glycol methacrylate, as a component 2.

2. The nail cosmetic material according to claim 1, wherein the component 2 includes at least two compounds selected from the group consisting of dimethyl acrylamide, diethyl acrylamide, acryloyl morpholine, and butoxy diethylene glycol methacrylate.

3. The nail cosmetic material according to claim 1 or 2, further comprising a photopolymerization initiator.

4. The nail cosmetic material according to any one of claims 1 to 3, further comprising a polymer and/or an oligomer.

5. The nail cosmetic material according to claim 4, wherein the polymer and/or the oligomer are a polymer and/or an oligomer having a urethane bond.

6. The nail cosmetic material according to claim 4 or 5, wherein the polymer and/or the oligomer have a (meth)acryloyl group.

7. The nail cosmetic material according to any one of claims 4 to 6, wherein the content of the polymer and/or the oligomer is 10 to 60 parts by mass with respect to 100 parts by mass of the nail cosmetic material.

8. The nail cosmetic material according to any one of claims 1 to 7, wherein the content of the component 1 is 1 to 25 parts by mass with respect to 100 parts by mass of the nail cosmetic material.

9. The nail cosmetic material according to any one of claims 1 to 8, wherein the content of the component 2 is 1 to 50 parts by mass with respect to 100 parts by mass of the nail cosmetic material.

10. The nail cosmetic material according to any one of claims 1 to 9, further comprising a solvent.

11. A nail art kit comprising the nail cosmetic material according to any one of claims 1 to 10.
